# EUROPEAN PATENT APPLICATION

(11) **EP 4 112 733 A1**
(43) Date of publication of application: **04.01.2023**
(21) Application number: 20921866.8
(22) Date of filing: 31.03.2020
(51) Int. Cl.: C12P 13/00, C12P 17/12, C12P 17/04, C12N 9/10

(54) **METHOD FOR SYNTHESIZING CHIRAL DIAMINE COMPOUND**

(30) Priority: 26.02.2020 CN 202010118060
(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville, North Carolina 27560 (US); XIAO, Yi, Tianjin 300457 (CN); ZHANG, Na, Tianjin 300457 (CN); LI, Xiang, Tianjin 300457 (CN); JIANG, Xiangjun, Tianjin 300457 (CN); ZHAO, Tong, Tianjin 300457 (CN)
(74) Representative: Berggren Oy
(86) International application number: PCT/CN2020/082594
(87) International publication number: WO 2021/168987

(57) **Abstract**

Provided is a method for synthesizing a chiral diamine compound. The synthesizing method includes: converting a substrate represented by Formula I into a chiral diamine compound in Formula I by using a transaminase, herein n=1~10, an R group represents an alkyl, a cycloalkyl, a heteroatom-containing alkyl, a heteroatom-containing cycloalkyl, a heteroatom-containing aryl, an amide compound residue, or an ether compound residue, and a hetero atom is at least one from among O, S and N; R1 and R2 are the same or not the same, the R1 and R2 are respectively and independently hydrogen, a C1-C3 alkyl, or an amino protecting group; and the transaminases are derived from a plurality of strains.The transaminases all have a relatively high reaction selectivity and activity on various substrates represented by Formula 1. By catalyzing and synthesizing a chiral diamine compound by using the biological enzyme, not only is the substrate scope wider, but the route also short and product yield high, thus greatly reducing production costs and reducing the production of an organic solvent and the three wastes.

## Description

### Technical Field

The present invention relates to the field of synthesis of chiral diamine compounds, in particular to a method for synthesizing a chiral diamine compound.

### Background

Chiral diamine compounds widely exist in many biologically active natural products and drug molecules, such as: an ornithine, a lysine, a vitamin H, and a drug levamisole. There is a diamine compound in a molecule of Oxaliplatin, it has certain antitumor activity. Compounds with diamine structures are also widely used in organic synthesis. As a synthesis building block, it may effectively construct a nitrogen heterocycle, and may also be used as a ligand complexed with a metal to generate a catalyst with high reaction activity. The diamine compounds are also widely used in the fields of liquid crystal materials, aerospace materials, microelectronics and the like. The chiral diamine compounds may also be used as a chiral resolving reagent to resolve an aldehyde enantiomer. Therefore, the synthesis of chiral diamines is always a research hotspot of chemists (Angew.Chem.Int.Ed.1998,37,2580).

At present, synthesis methods for the chiral diamine compounds include asymmetric strecker reaction (Chem.Rev., 2011, 111, 6947), asymmetric Michel addition (CN 105367427 A), aziridine asymmetric ring opening (CN 105753752 A) and other methods, but these methods have different degrees of limitations in the scope of application and practicability of substrates, and these methods all synthesize 1,2-diamine compounds. The asymmetric Michel addition and the aziridine asymmetric ring opening usually require expensive metals and chiral catalysts, this greatly limit an application of a reaction system. In addition, highly toxic azide compounds or cyanating reagents may be used in the reaction, and the amount of three wastes in a chemical route is large, and it is difficult to treat.

Therefore, how to improve the existing methods to expand the scope of application to the substrate becomes an urgent technical problem to be solved.

### Summary

A main purpose of the present invention is to provide a method for synthesizing a chiral diamine compound, as to solve a problem in an existing technology that a method has limitations on scope of application to a substrate.

In order to achieve the above purpose, the present invention provides a method for synthesizing a chiral diamine compound, and the synthesis method includes: converting a substrate shown in Formula I into a chiral diamine compound by using a transaminase:

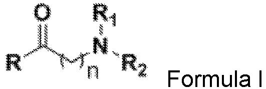

Herein, n=1~10, the R group represents an alkyl, a cycloalkyl, a heteroatom-containing alkyl, a heteroatom-containing cycloalkyl, a heteroatom-containing aryl, an amide compound residue or an ether compound residue, herein, the heteroatom is at least one of O, S and N; R1 and R2 are the same or different, and R1 and R2 are each independently hydrogen, a C1-C3 alkyl or an amino protecting group; the transaminase is derived from Chromobacterium violaceum DSM30191(CVTA)(NCBI Reference Sequence:WP_011135573.1), Fonsecaea pedrosoi CBS 271.37(NCBI Reference Sequence:XP_013286281.1), Klebsiella pneumoniae subsp.pneumoniae EcI8(GenBank:CCN29541.1), Mycobacterium goodii(GenBank:AKS36000.1), Paracoccus denitrificans(NCBI Reference Sequence:WP_011746975.1), Penicillium brasilianum(GenBank:CEJ55334.1), Enterobacter sp.TL3(NCBI Reference Sequence:WP_014885677.1), Aspergillus terreusNIH2624(NCBI Reference Sequence:XP_001209325.1), Exophiala spinifera(NCBI Reference Sequence:XP_016233821.1), Deinococcus geothermalis(strain DSM 11300)(NCBI Reference Sequence:WP_011530545.1), Geomyces destructans 20631-21(GdTA)in E.coli(GenBank:ELR05573.1), Pseudomonas putida KT2440(NCBI Reference Sequence:WP_010954554.1), Lysinibacillus sphaericus(NCBI Reference Sequence:WP_024363741.1), Bacillus megaterium DSM 319(NCBI Reference Sequence:WP_013082219.1), Trichoderma harzianum(GenBank:KKP07030.1), Aspergillus fumigatus R-ATAs(AspFum)(NCBI Reference Sequence:XP_748821.1), Geobacillus thermodenitrificans subsp.thermodenitrificans DSM 465(NCBI Reference Sequence:WP_008879436.1), Cladophialophora bantiana CBS 173.52(NCBI Reference Sequence:XP_016617948.1), Bacillus megaterium(NCBI Reference Sequence:WP_016763026.1), Burkholderia thailandensis MSMB121(BtS-TA)(GenBank:AGK49399.1), Klebsiella pneumoniae subsp.pneumoniae MGH 78578(NCBI Reference Sequence:WP_002920226.1), Geobacillus toebii(NCBI Reference Sequence:WP_062753894.1) and Talaromyces cellulolyticus(GenBank:GAM37533.1).

Further, the transaminase has an amino acid sequence shown in SEQ ID NO: 1, SEQ ID NO: 2, SEQ ID NO: 3 or SEQ ID NO: 4.

Further, the amino protecting group is selected from any one of tert-butoxycarbonyl, benzyloxycarbonyl, formyl, trifluoroacetyl, benzyl, trityl and 9-fluorenylmethoxycarbonyl.

Further, the substrate is selected from any one of the followings:

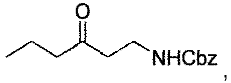

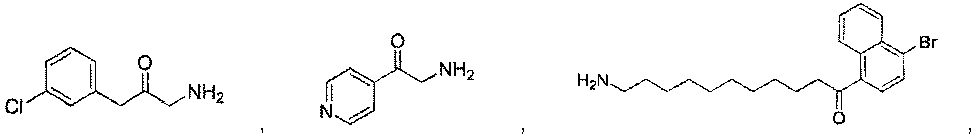

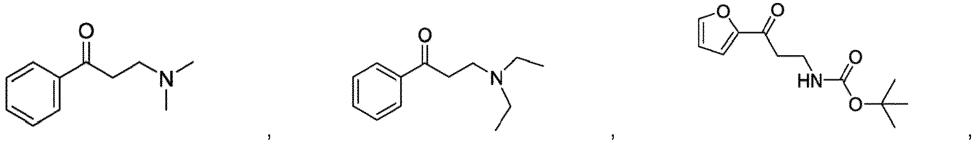

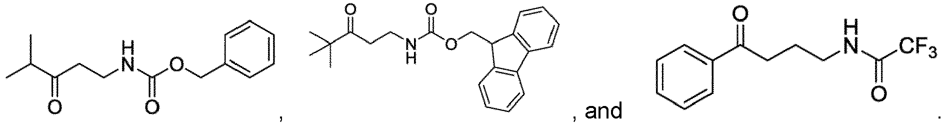

Further, the synthesis method includes: mixing a phosphate buffer with an amino donor, to obtain a first mixed solution, adding a substrate represented by Formula I to the first mixed solution, to obtain a second mixed solution; adding a transaminase to the second mixed solution, to obtain a reaction mixture; and isolating the chiral diamine compound from the reaction mixture.

Further, before adding the substrate to the first mixed solution, the synthesis method further includes: adjusting a pH value of the first mixed solution to 7.0-9.0; and preferably, adding the transaminase to the second mixed solution, and adjusting pH value of the second mixed solution after adding the transaminase to 7.0-9.0, to obtain the reaction mixture.

Further, the step of isolating the chiral diamine compound from the reaction mixture includes: adjusting the acidity of the reaction mixture to denature the transaminase, and preferably adjusting a pH value of the reaction mixture to 1-2; filtering and removing the denatured transaminase, to obtain a preliminary filtrate; adjusting a pH value of the preliminary filtrate to alkaline, to obtain an alkaline filtrate; extracting the alkaline filtrate, to obtain the chiral diamine compound; preferably, a pH value of the alkaline filtrate is 12~13; preferably, the extraction is performed for many times, more preferably 2~5 times, and more preferably, the first extraction is performed with dichloromethane, and the remaining times of the extraction are performed with the dichloromethane or ethyl acetate subsequently.

Further, after the extractions, a step of drying an organic phase obtained by the extractions is also included. Preferably, the organic phases obtained by each time of the extraction are combined, to obtain an extract; the extract is dried, to obtain a dry organic phase product; and the dry organic phase product is placed under conditions of temperature <45°C and pressure ≤-0.08 Mpa and concentrated until there is no fraction, to obtain the chiral diamine compound.

Further, a mass ratio of the transaminase to the substrate is 0.4:1~1.0:1.

Further, the concentration of the substrate is 60 g/L~100 g/L.

Further, the amino donor is selected from isopropylamine, isopropylamine hydrochloride, alanine, phenethylamine or n-butylamine.

A technical scheme of the present invention is applied, the transaminase used has specificity on the substrate represented in Formula I, and may effectively catalyze this type of the substrate to be converted into the chiral diamine compound. In addition, the transaminase is relatively high in reaction selectivity and activity to a plurality of the substrates represented in Formula I. Therefore, the use of this biological enzyme to catalyze the synthesis of the chiral diamine compound is not only suitable for a wider range of the substrate (all 1,2-diamine compounds are obtained by the reaction in an existing technology, but 1,3-diamine, 1,4-diamine and other compounds may also be synthesized in the present application), and the synthesis method is short in route, and high in product yield, so the production cost is greatly reduced, and the generation of organic solvents and three wastes is reduced.

### Brief Description of the Drawings

Drawings of the description constituting a part of the present application are used to provide further understanding of the present invention, and exemplary embodiments of the present invention and descriptions thereof are used to explain the present invention, and do not constitute improper limitation to the present invention. In the drawings:
Fig. 1 shows effects of different enzyme amounts on the conversion rate of the same amount of a substrate in Embodiment 11 of the present invention.
Fig. 2 shows effects of the same enzyme amount on the conversion rate of the substrates with different concentrations in Embodiment 12 of the present invention.
Fig. 3 shows effects of different amino donors on the conversion rate of the substrate in the same reaction in Embodiment 13 of the present invention.
Fig. 4 shows that transaminases from different sources in Embodiment 14 of the present invention all have conversion activities for the same substrate.

### Detailed Description of the Embodiments

It should be noted that embodiments in the present application and features of the embodiments may be combined with each other in the case without conflicting. The present invention is described in detail below in combination with the embodiments.

As mentioned in the background, in an existing technology, there is a problem that a method for synthesizing a chiral diamine compound has limitations on a scope of application of a substrate. In order to improve this situation, the inventor of the present application tries to improve an existing synthesis route from the perspective of high efficiency and green and environmental protection. In the research process, it is found by the inventor that a transaminase developed by the applicant himself not only has the general catalytic activity of the transaminase for catalyzing ketone compounds with a pyridoxal phosphate (PLP) as a coenzyme, but also has the activity of catalyzing a plurality of different substrates and converting it into the chiral diamine compound. In addition, it is found from the further research that while the transaminase is used to catalyze the chiral diamine compound, not only a target product may be obtained by a one-step reaction, but also the substrate type is wide. Due to the high selectivity of a biotransformation reaction using the transaminase, an enantiomeric excess (ee) value of the target product is greatly improved. Moreover, the use of a biological enzyme to catalyze the reaction may improve the amount of the substrate, greatly improve the production efficiency and reduce the generation of organic solvents and three wastes.

On the basis of the above research results, the applicant proposes a technical scheme of the present application. In a typical embodiment of the present application, a method for synthesizing a chiral diamine compound is provided, and the synthesis method includes: converting a substrate represented by Formula I into a chiral diamine compound by using a transaminase:

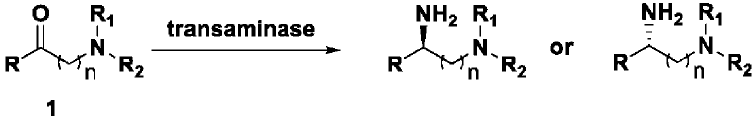

Herein, n=1~10, an R group represents an alkyl, a cycloalkyl, a heteroatom-containing alkyl, a heteroatom-containing cycloalkyl, a heteroatom-containing aryl, an amide compound residue or an ether compound residue; R1, and R2 are the same or different, and R1 and R2 are each independently hydrogen, a C1-C3 alkyl or an amino protecting group, and the transaminase is derived from Chromobacterium violaceum DSM30191(CVTA)(NCBI Reference Sequence:WP_011135573.1), Fonsecaea pedrosoi CBS 271.37(NCBI Reference Sequence:XP_013286281.1), Klebsiella pneumoniae subsp.pneumoniae EcI8(GenBank:CCN29541.1), Mycobacterium goodii(GenBank:AKS36000.1), Paracoccus denitrificans(NCBI Reference Sequence:WP_011746975.1), Penicillium brasilianum(GenBank:CEJ55334.1), Enterobacter sp.TL3(NCBI Reference Sequence:WP_014885677.1), Aspergillus terreusNIH2624(NCBI Reference Sequence:XP_001209325.1), Exophiala spinifera(NCBI Reference Sequence:XP_016233821.1), Deinococcus geothermalis(strain DSM 11300)(NCBI Reference Sequence:WP_011530545.1), Geomyces destructans 20631-21 (GdTA)in E.coli(GenBank:ELR05573.1), Pseudomonas putida KT2440(NCBI Reference Sequence:WP_010954554.1), Lysinibacillus sphaericus(NCBI Reference Sequence:WP_024363741.1), Bacillus megaterium DSM 319(NCBI Reference Sequence:WP_013082219.1), Trichoderma harzianum(GenBank:KKP07030.1), Aspergillus fumigatus R-ATAs(AspFum)(NCBI Reference Sequence:XP_748821.1), Geobacillus thermodenitrificans subsp.thermodenitrificans DSM 465(NCBI Reference Sequence:WP_008879436.1), Cladophialophora bantiana CBS 173.52(NCBI Reference Sequence:XP_016617948.1), Bacillus megaterium(NCBI Reference Sequence:WP_016763026.1), Burkholderia thailandensis MSMB121(BtS-TA)(GenBank:AGK49399.1), Klebsiella pneumoniae subsp.pneumoniae MGH 78578(NCBI Reference Sequence:WP_002920226.1), Geobacillus toebii(NCBI Reference Sequence:WP_062753894.1) and Talaromyces cellulolyticus(GenBank:GAM37533.1).

Preferably, the transaminase has an amino acid sequence shown respectively in SEQ ID NO: 1, SEQ ID NO: 2, SEQ ID NO: 3 or SEQ ID NO: 4:

MQKQRTCSQWRELDAAHHLHPFTDTASLNQAGARVMTRGEGVYLWDCEGNKIIDGMAGLW
CVNVGYGRKDFAEAARRQMEELPFYNTFFKTTHPAVVELSSLLAEVTPAGFDRVFYTNSGSESVD
TMIRMVRRYWDVQGKPEKKTLIGRWNGYHGSTIGGASLGGMKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMVLAFTLV
KNKAKRELFPDFGEIGTLCRDIFFRNNLIMTACGDHIVSAPPLVMTRAEVDEMLAVAERCLEEFEQT
LKARGLA (SEQ ID NO:1).

MQKQRTCSQWRELDAAHHLHPFTDTASLNQAGARVMTRGEGVYLWDCEGNKIIDGMAGLW
CVNVGYGRKDFAEAARRQMEELPFYNTFYKTTHPAVVELSSLLAEVTPAGFDRVFYTNSGSESVD
TMIRMVRRYWDVQGKPEKKTLIGRWWGYHGSTIGGASLGGFKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFFRNNLIMDSCGDHIVAAPPLVMTRAEVDEMLAVAERCLEEFEQTL

KARGLA (SEQ ID NO:2).

MQKQRTCSQWRELDAAHHLHPFTDTASLNQAGARVMTRGEGVYLWDCEGNKIIDGMAGLW
CVNVGYGRKDFAEAARRQMEELPFYNTFYKTTHPAVVELSSLLAEVTPAGFDRVFYTNSGSESVD
TMIRMVRRYWDVQGKPEKKTLIGRWWGYHGSTIGGASLGGFKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFQRNNLIMDSCGDHIVSAPPLVMTRAEVDEMLAVAERCLEEFEQTL
KARGLA (SEQ ID NO:3).

MQKQRTCSQWRELDAAHHLHPFTDTASLNQAGARVMTRGEGVYLWDCEGNKIIDGMAGLW
CVNVGYGRKDFAEAARRQMEELPFYNTFYKTTHPAVVELSSLLAEVTPAGFDRVFYTNSGSESVD
TMIRMVRRYWDVQGKPEKKTLIGRWWGYHGSTIGGASLGGFKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFHRNNLIMDSCGDHIVSAPPLVMTRAEVDEMLAVAERCLEEFEQTL
KARGLA (SEQ ID NO:4).

As described above, the above transaminase of the present application has substrate specificity on the substrate represented by Formula I, and may effectively catalyze this type of the substrate to be converted into the chiral diamine compound. In addition, the transaminase is relatively high in reaction selectivity and activity to a plurality of the substrates represented by Formula I. Therefore, the use of this biological enzyme to catalyze the synthesis of the chiral diamine compound is not only suitable for a wider range of the substrate (all 1,2-diamine compounds are obtained by the reaction in an existing technology, but 1,3-diamine, 1,4-diamine and other compounds may also be synthesized in the present application), and the synthesis method is short in route, and high in product yield, so the production cost is greatly reduced, and the generation of organic solvents and three wastes is reduced.

The heteroatom in the "heteroatom-containing alkyl, heteroatom-containing cycloalkyl, and heteroatom-containing aryl" in the above substrates may be at least one of O, S and N.

The above transaminases with the amino acid sequences shown in SEQ ID NO: 1, SEQ ID NO: 2, SEQ ID NO: 3 and SEQ ID NO: 4 are all derived from ω-transaminase, and Chromobacterium violaceum DSM30191 (CVTA).

The above amino protecting groups include, but are not limited to, tert-butoxycarbonyl, benzyloxycarbonyl, formyl, trifluoroacetyl, benzyl, trityl or 9-fluorenylmethoxycarbonyl.

In a preferred embodiment, the substrate shown in Formula I is selected from any one of the followings: 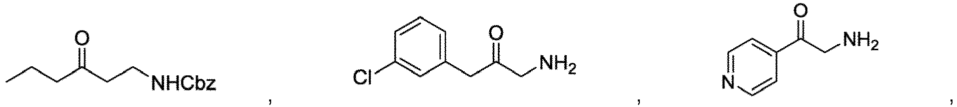 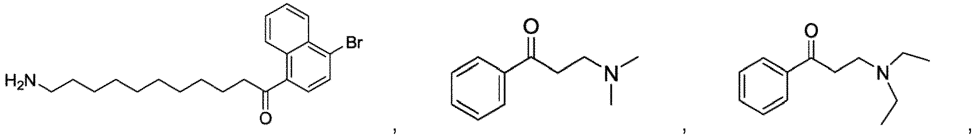 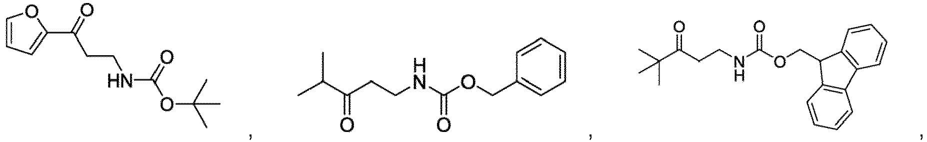 and

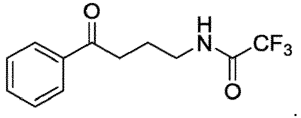

The transaminase of the present application has the catalytic activity and stereoselectivity for the above substrates shown in Formula I.

A specific step of using the transaminase of the present application to catalyze a transamination reaction of the substrate shown in Formula I is similar to an existing transamination reaction. In a preferred embodiment, the above synthesis method includes: mixing a phosphate buffer with an amino donor, to obtain a first mixed solution, adding a substrate represented by Formula I to the first mixed solution, to obtain a second mixed solution; adding a transaminase to the second mixed solution, to obtain a reaction mixture; and isolating the chiral diamine compound from the reaction mixture.

The synthesis method reduces the reaction volume by the above step-by-step reaction, so that the transaminase is relatively high in catalytic activity and efficiency on the substrate, thereby the higher product yield is obtained, and the production efficiency is improved.

In a preferred embodiment, before adding the substrate shown in Formula I to the first mixed solution, the synthesis method further includes: adjusting a pH value of the first mixed solution to 7.0-9.0; and preferably, adding the transaminase to the second mixed solution, and adjusting pH value of the second mixed solution after adding the transaminase to 7.0-9.0, to obtain the reaction mixture.

In the above preferred embodiment, before adding the substrate shown in Formula I to the first mixed solution, the pH value of the first mixed solution is adjusted, its purpose is to adjust to the optimum pH of the enzyme reaction before adding the enzyme, and prevent the inactivation of the enzyme in the adding process. After the transaminase is added to the second mixed solution, the pH value of the system is also adjusted, its function is to ensure that the reaction is performed at the optimum pH. It is better to keep the same range of pH value adjustment for the above two times, for example, it is previously adjusted to 7.8, and it is also adjusted to 7.8 after the transaminase is added.

The above step of isolating the target product from the mixture after the reaction may be performed by using a known isolation and purification method. In a preferred embodiment, the step of isolating the chiral diamine compound from the reaction mixture includes: adjusting the acidity of the reaction mixed solution to denature the transaminase, and preferably adjusting a pH value of the reaction mixed solution to 1-2; filtering and removing the denatured transaminase, to obtain preliminary filtrate; adjusting a pH value of the preliminary filtrate to alkaline, to obtain alkaline filtrate; extracting the alkaline filtrate, to obtain the chiral diamine compound; preferably, a pH value of the alkaline filtrate is 12~13; preferably, the extraction is performed for many times, more preferably 2~5 times, and more preferably, the first extraction is performed with dichloromethane, and the remaining times of the extraction are performed with the dichloromethane or ethyl acetate subsequently.

A mode of adjusting the acidity is used to denature an enzyme protein after the reaction. Compared with other denaturation modes (such as high temperature, alkali adjustment or salting out), it has advantages that the denaturation is more thorough, most of products are stable under an acidic condition, and a subsequent operation may directly extract other impurities in the system under the acidic condition. The purpose of the first extraction with dichloromethane is to remove impurities that can be taken in the system under the acidic conditions. The extraction with the dichloromethane first has the advantage of higher extraction efficiency compared to the extraction with other extraction solvents (such as ethyl acetate, methyl tert-butyl ether, or isopropyl acetate).

In order to further improve the purity and yield, in a preferred embodiment, after the extraction, a step of drying an organic phase obtained by the extraction is also included. Preferably, the organic phases obtained by each time of the extraction are combined, to obtain an extract; the extract is dried, to obtain a dry organic phase product; and the dry organic phase product is placed under conditions of temperature <45 °C and pressure ≤-0.05 Mpa and concentrated until there is no fraction (a purpose of drying is to reduce the water content in the product, and the pressure affects the concentration rate).

In a preferred embodiment, the mass ratio of the transaminase to the substrate shown in Formula I is: 0.4:1∼1.0:1, the reaction is performed according to this mass ratio, and the conversion rate of the substrate may reach more than 92%.

In a preferred embodiment, the concentration of the substrate shown in Formula I is 60 g/L~100 g/L. In this concentration range, the use of the biological enzyme for the reaction not only has the conversion rate of up to 95% of the substrate, but also helps to improve the production efficiency, and reduce the generation of organic solvents and three wastes.

In a preferred embodiment, the amino donor is selected from isopropylamine, isopropylamine hydrochloride, alanine, phenethylamine or n-butylamine.

The beneficial effects of the present application are further described below in combination with specific embodiments. It should be noted that the room temperature in the following embodiments refers to a temperature within the normal temperature range of 10~25°C, and the transaminase used in the following Embodiments 1 to 15 is the transaminase shown in SEQ ID NO: 4.

### Embodiment 1

50 mL of 100 mmol/L phosphate buffer (5 vol) and 24 mL of 5 mol/L isopropylamine hydrochloride solution (2.4 vol, 3 eq) are added to a 250 mL four-neck flask at the room temperature, and it is adjusted that pH=7.5~8.0. Then 0.1 g of pyridoxal phosphate (1wt%), and 10 g of 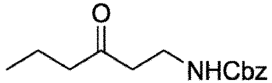 are added, and stirred uniformly, then 10 ml of transaminase enzyme solution (0.5 wt, 0.5 g/ml) is added to adjust pH=7.5~8.0. The temperature is raised to 30°C and it is stirred and reacted overnight. After the reaction is completed, the system is adjusted to pH=1, as to denature a protein. After being filtered, filtrate is extracted with 50 mL of dichloromethane. An aqueous phase is adjusted to pH=12, and extracted for three times with 50 ml of the dichloromethane. Organic phases are combined and dried with an anhydrous magnesium sulfate, then the organic phase is concentrated until there is no fraction under conditions of T<35 °C and P≤-0.06Mpa. A target product 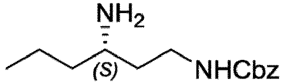 is obtained.

It is detected by a high performance liquid chromatography (HPLC), purity>98%, ee value>99%, yield: 90%.

### Embodiment 2

60 mL of 100 mmol/L phosphate buffer (6 vol) and 18 mL of 5 mol/L isopropylamine hydrochloride solution (1.8 vol, 3 eq) are added to a 250 mL four-neck flask at the room temperature, and it is adjusted that pH=8.0~8.5. Then 0.1 g of pyridoxal phosphate (1wt%), and 10 g of 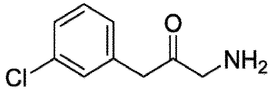 are added, and stirred uniformly, then 10 ml of transaminase enzyme solution (0.5 wt, 0.5 g/ml) is added to adjust pH=8.0~8.5. The temperature is raised to 20°C and it is stirred and reacted overnight. After the reaction is completed, the system is adjusted to pH=2, as to denature a protein. After being filtered, filtrate is extracted with 50 mL of dichloromethane. An aqueous phase is adjusted to pH=13, and extracted for three times with 50 ml of the dichloromethane. Organic phases are combined and dried with an anhydrous magnesium sulfate, then the organic phase is concentrated until there is no fraction under conditions of T<45 °C and P≤-0.08Mpa. A target product 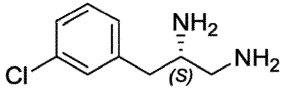 is obtained.

It is detected by HPLC, purity>92%, ee value>99%, yield: 86%.

### Embodiment 3

70 mL of 100 mmol/L phosphate buffer (7 vol) and 13 mL of 5 mol/L isopropylamine hydrochloride solution (1.3 vol, 3 eq) are added to a 250 mL four-neck flask at the room temperature, and it is adjusted that pH=7.0~7.5. Then 0.1 g of pyridoxal phosphate (1wt%), and 10 g of 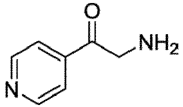 are added, and stirred uniformly, then 10 ml of transaminase enzyme solution (0.5 wt, 0.5 g/ml) is added. The temperature is raised to 50°C and it is stirred and reacted overnight. After the reaction is completed, the system is adjusted to pH=1, as to denature a protein. After being filtered, filtrate is extracted with 50 mL of dichloromethane. An aqueous phase is adjusted to pH=13, and extracted for three times with 50 ml of the dichloromethane. Organic phases are combined and dried with an anhydrous magnesium sulfate, then the organic phase is concentrated until there is no fraction under conditions of T<35 °C and P≤-0.06Mpa. A target product 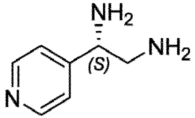 is obtained.

It is detected by HPLC, purity>97%, ee value>99%, yield: 78%.

### Embodiment 4

50 mL of 100 mmol/L phosphate buffer (5 vol) and 38 mL of 5 mol/L isopropylamine hydrochloride solution (3.8 vol, 3 eq) are added to a 250 mL four-neck flask at the room temperature, and it is adjusted that pH=8.5~9.0. Then 0.1 g of pyridoxal phosphate (1wt%), and 10 g of 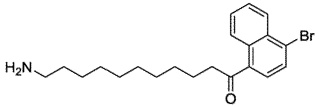 are added, and stirred uniformly, then 10 ml of transaminase enzyme solution (0.5 wt, 0.5 g/ml) is added to adjust pH=8.5~9.0. The temperature is raised to 10°C and it is stirred and reacted overnight. After the reaction is completed, the system is adjusted to pH=2, as to denature a protein. After being filtered, filtrate is extracted with 50 mL of dichloromethane. An aqueous phase is adjusted to pH=13, and extracted for three times with 50 ml of the dichloromethane. Organic phases are combined and dried with an anhydrous magnesium sulfate, then the organic phase is concentrated until there is no fraction under conditions of T<35 °C and P≤-0.06Mpa. A target product 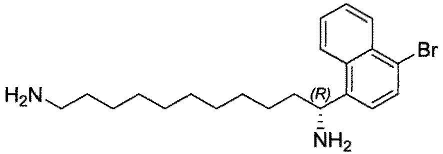 is obtained.

It is detected by HPLC, purity>95%, ee value>99%, yield: 79%.

### Embodiment 5

70 mL of 100 mmol/L phosphate buffer (7 vol) and 17 mL of 5 mol/L isopropylamine hydrochloride solution (1.7 vol, 3 eq) are added to a 250 mL four-neck flask at the room temperature, and it is adjusted that pH=7.5~8.0. Then 0.1 g of pyridoxal phosphate (1wt%), and 10 g of 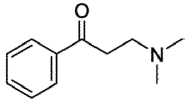 are added, and stirred uniformly, then 10 ml of transaminase enzyme solution (0.5 wt, 0.5 g/ml) is added to adjust pH=7.5~8.0. The temperature is raised to 29°C and it is stirred and reacted overnight. After the reaction is completed, the system is adjusted to pH=1, as to denature a protein. After being filtered, filtrate is extracted with 50 mL of dichloromethane. An aqueous phase is adjusted to pH=12, and extracted for three times with 50 ml of ethyl acetate. Organic phases are combined and dried with an anhydrous magnesium sulfate, then the organic phase is concentrated until there is no fraction under conditions of T<45 °C and P≤-0.08Mpa. A target product 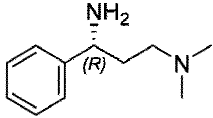 is obtained.

It is detected by HPLC, purity>98%, ee value>99%, yield: 87%.

### Embodiment 6

140 mL of 100 mmol/L phosphate buffer (7 vol) and 50 mL of 5 mol/L isopropylamine hydrochloride solution (1.7 vol, 3 eq) are added to a 500 mL four-neck flask at the room temperature, and it is adjusted that pH=7.5~8.0. Then 0.2 g of pyridoxal phosphate (1wt%), and 20 g of 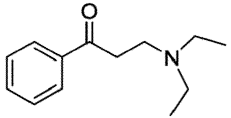 are added, and stirred uniformly, then 20 ml of transaminase enzyme solution (0.5 wt, 0.5 g/ml) is added to adjust pH=7.5~8.0. The temperature is raised to 32°C and it is stirred and reacted overnight. After the reaction is completed, the system is adjusted to pH=1, as to denature a protein. After being filtered, filtrate is extracted with 100 mL of dichloromethane. An aqueous phase is adjusted to pH=12, and extracted for three times with 100 ml of the ethyl acetate. Organic phases are combined and dried with an anhydrous magnesium sulfate, then the organic phase is concentrated until there is no fraction under conditions of T<45 °C and P≤-0.08Mpa. A target product 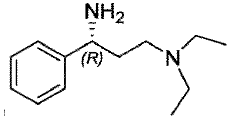 is obtained.

It is detected by HPLC, purity>97%, ee value>99%, yield: 84%.

### Embodiment 7

220 mL of 100 mmol/L phosphate buffer (11 vol) and 50 mL of 5 mol/L isopropylamine hydrochloride solution (2.5 vol, 3 eq) are added to a 500 mL four-neck flask at the room temperature, and it is adjusted that pH=7.5~8.0. Then 0.2 g of pyridoxal phosphate (1wt%), and 20 g of 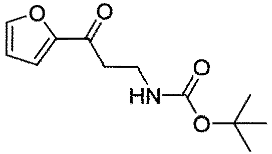 are added, and stirred uniformly, then 40 ml of transaminase enzyme solution (1.0 wt, 0.5 g/ml) is added to adjust pH=7.5~8.0. The temperature is raised to 32°C and it is stirred and reacted overnight. After the reaction is completed, the system is adjusted to pH=1, as to denature a protein. After being filtered, filtrate is extracted with 100 mL of dichloromethane. An aqueous phase is adjusted to pH=12, and extracted for three times with 100 ml of the ethyl acetate. Organic phases are combined and dried with an anhydrous magnesium sulfate, then the organic phase is concentrated until there is no fraction under conditions of T<45 °C and P≤-0.08Mpa. A target product 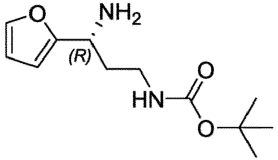 is obtained.

It is detected by HPLC, purity>95%, ee value>99%, yield: 85%.

### Embodiment 8

180 mL of 100 mmol/L phosphate buffer (12 vol) and 36 mL of 5 mol/L isopropylamine hydrochloride solution (2.4 vol, 3 eq) are added to a 500 mL four-neck flask at the room temperature, and it is adjusted that pH=7.5~8.0. Then 0.15 g of pyridoxal phosphate (1wt%), and 15 g of 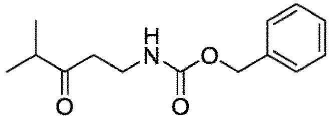 are added, and stirred uniformly, then 30 ml of transaminase enzyme solution (1.0 wt, 0.5 g/ml) is added to adjust pH=7.5~8.0. The temperature is raised to 35°C and it is stirred and reacted overnight. After the reaction is completed, the system is adjusted to pH=1, as to denature a protein. After being filtered, filtrate is extracted with 100 mL of dichloromethane. An aqueous phase is adjusted to pH=12, and extracted for three times with 100 ml of ethyl acetate. Organic phases are combined and dried with an anhydrous magnesium sulfate, then the organic phase is concentrated until there is no fraction under conditions of T<45 °C and P≤-0.08Mpa. A target product 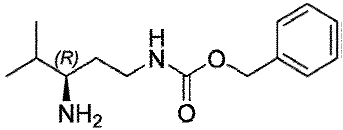 is obtained.

It is detected by HPLC, purity>96%, ee value>99%, yield: 91%.

### Embodiment 9

180 mL of 100 mmol/L phosphate buffer (12 vol) and 26 mL of 5 mol/L isopropylamine hydrochloride solution (1.7 vol, 3 eq) are added to a 500 mL four-neck flask at the room temperature, and it is adjusted that pH=7.5~8.0. Then 0.15 g of pyridoxal phosphate (1wt%), and 15 g of 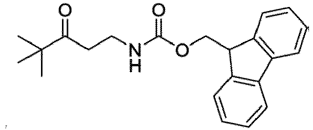 are added, and stirred uniformly, then 30 ml of transaminase enzyme solution (1.0 wt, 0.5 g/ml) is added to adjust pH=7.5~8.0. The temperature is raised to 40°C and it is stirred and reacted overnight. After the reaction is completed, the system is adjusted to pH=1, as to denature a protein. After being filtered, filtrate is extracted with 100 mL of dichloromethane. An aqueous phase is adjusted to pH=12, and extracted for three times with 100 ml of ethyl acetate. Organic phases are combined and dried with an anhydrous magnesium sulfate, then the organic phase is concentrated until there is no fraction under conditions of T<45 °C and P≤-0.08Mpa. A target product 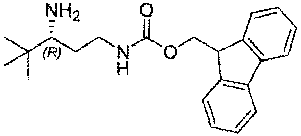 is obtained.

It is detected by HPLC, purity>97%, ee value>99%, yield: 90%.

### Embodiment 10

110 mL of 100 mmol/L phosphate buffer (11 vol) and 23 mL of 5 mol/L isopropylamine hydrochloride solution (2.3 vol, 3 eq) are added to a 500 mL four-neck flask at the room temperature, and it is adjusted that pH=7.5~8.0. Then 0.1 g of pyridoxal phosphate (1wt%), and 10 g of 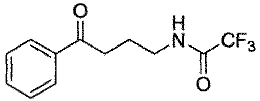 are added, and stirred uniformly, then 10 ml of transaminase enzyme solution (0.5 wt, 0.5 g/ml) is added to adjust pH=7.5~8.0. The temperature is raised to 40°C and it is stirred and reacted overnight. After the reaction is completed, the system is adjusted to pH=2, as to denature a protein. After being filtered, filtrate is extracted with 50 mL of dichloromethane. An aqueous phase is adjusted to pH=12, and extracted for three times with 50 ml of ethyl acetate. Organic phases are combined and dried with an anhydrous magnesium sulfate, then the organic phase is concentrated until there is no fraction under conditions of T<45 °C and P≤-0.08Mpa. A target product 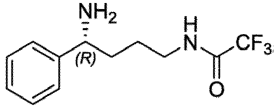 is obtained.

It is detected by HPLC, purity>95%, ee value>99%, yield: 83%.

### Embodiment 11

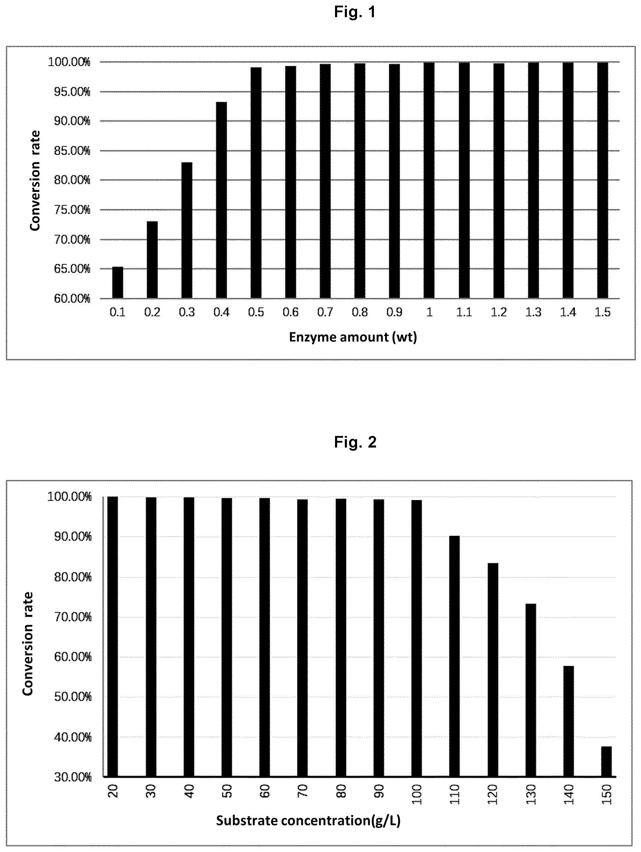

Embodiment 2 is taken as an example, the enzyme amount of the reaction is optimized and a specific operation is as follows: 1.2 mL of 100 mmol/L phosphate buffer (6 vol), and 0.36 ml of 5 mol/L isopropylamine hydrochloride solution (1.8 vol, 3eq) are added to a 10 mL vial at a room temperature, it is adjusted that pH=8.0~8.5. Then 0.002 g of pyridoxal phosphate (1wt%), and 0.2 g of 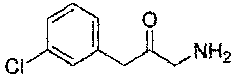 are added, and mixed uniformly, then 0.04 ml, 0.08 ml, 0.12 ml, 0.16 ml, 0.2 ml, 0.24 ml, 0.28 ml, 0.32 ml, 0.36 ml, 0.4 ml, 0.44 ml, 0.48 ml, 0.52 ml, 0.56 ml, and 0.6 ml (the concentration of the enzyme solution is 0.5 g/ml, the corresponding mass ratios are 0.1 wt, 0.2 wt, 0.3 wt, 0.4 wt, 0.5 wt, 0.6 wt, 0.7 wt, 0.8 wt, 0.9 wt, 1.0 wt, 1.1 wt, 1.2 wt, 1.3 wt, 1.4 wt, and 1.5 wt) of transaminase enzyme solution are respectively added to adjust pH=8.0~8.5. The rotation speed of a shaker is 170 rpm, and the temperature is raised to 29°C and it is reacted overnight. Conversion rate results are shown in Fig. 1, and it is proved that the conversion rate of 0.5~1.0 wt substrate is greater than 99%, and there is no significant effect while the enzyme amount continues to increase.

### Embodiment 12

Embodiment 2 is taken as an example, the substrate concentration is optimized and a specific operation is as follows: 0.58 ml, 0.66 ml, 0.78 ml, 0.9 ml, 1.06 ml, 1.24 ml, 1.44 ml, 1.74 ml, 2.1 ml, 2.56 ml, 3.24 ml, 4.24 ml, 5.84 ml, and 9.24 ml of 100mmol/L phosphate buffer (2.9 vol, 3.3 vol, 3.9 vol, 4.5 vol, 5.3 vol, 6.2 ol, 7.2 vol, 8.7 vol, 10.5 vol, 12.8 vol, 16.2 vol, 21.2 vol, 29.2 vol, and 46.2 vol respectively), and 0.36 ml of 5 mol/L isopropylamine hydrochloride solution (1.8 vol, 3 eq) are respectively added to a 10-50 mL vial (an appropriate shake flask is selected according to the reaction volume) at the room temperature, it is adjusted that pH =8.0~8.5. Then 0.002 g of pyridoxal phosphate (1wt%), and 0.2 g (1vol, 0.2ml) of 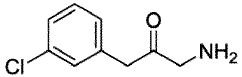 are added, and mixed uniformly, then 0.2 ml of transaminase enzyme solution (1 vol, 0.5 wt, the enzyme solution concentration is 0.5 g/ml) is added to adjust pH=8.0~8.5. The rotation speed of a shaker is 170 rpm, and the temperature is raised to 29°C and it is reacted overnight. As shown in Fig. 2, it is proved that the substrate conversion rate is greater than 99% at the concentration of 60-100 g/L. While the substrate concentration continues to decrease, the reaction volume may be increased, the amount of three wastes is increased, and there is no significant effect of further improvement of the conversion rate.

### Embodiment 13

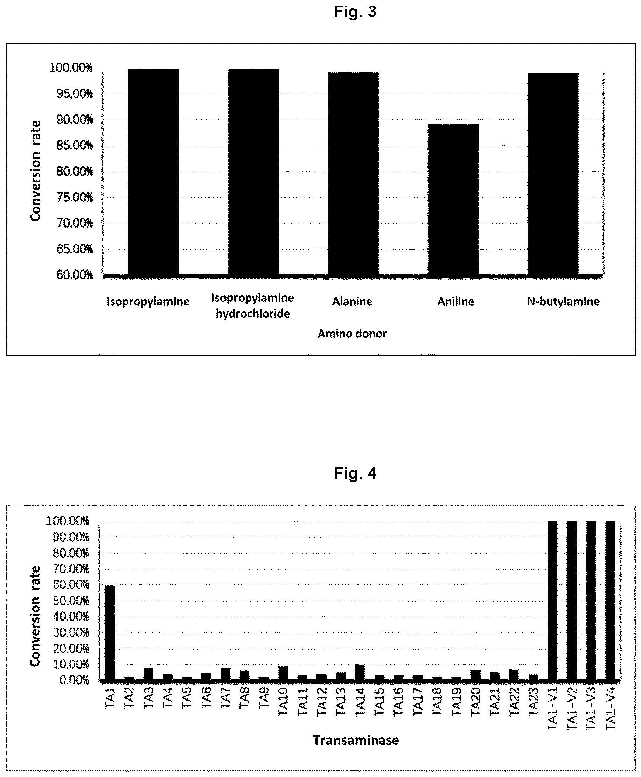

Embodiment 2 is taken as an example, the amino donor is optimized and a specific operation is as follows: 1.2 ml of 100 mmol/L phosphate buffer (6 vol) is added to a 10 mL vial at the room temperature, and systems of 3 eq amino donors (the amino donors are isopropylamine, isopropylamine hydrochloride, alanine, aniline, and n-butylamine respectively) are used respectively, and it is adjusted that pH=8.0~8.5. Then 0.002 g of pyridoxal phosphate (1wt%), and 0.2 g of 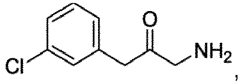 are added, and mixed uniformly, then 0.2 ml of transaminase enzyme solution (0.5 wt, the enzyme solution concentration is 0.5 g/ml) is added to adjust pH=8.0~8.5. The rotation speed of a shaker is 170 rpm, and the temperature is raised to 29°C and it is reacted overnight. Conversion rate results are shown in Fig. 3, and it is proved that the isopropylamine, isopropylamine hydrochloride, alanine, and n-butylamine are used as the amino donors, and the substrate conversion rate is greater than 99%. While the aniline is used as the amino donor, the conversion rate is only 89% under this reaction condition.

### Embodiment 14

The substrate of Embodiment 1 is taken as an example, the transaminase derived from Chromobacterium violaceum DSM30191 (CVTA) (NCBI Reference Sequence: WP_011135573.1) is numbered as TA1, the transaminase derived from Fonsecaea pedrosoi CBS 271.37 (NCBI Reference Sequence: XP_013286281.1) is numbered as TA2, the transaminase derived from Klebsiella pneumoniae subsp.pneumoniae Ecl8 (GenBank: CCN29541.1) is numbered as TA3, the transaminase derived from Mycobacterium goodii (GenBank: AKS36000.1) is numbered as TA4, the transaminase derived from Paracoccus denitrificans (NCBI Reference Sequence: WP_011746975.1) is numbered as TA5, the transaminase derived from Penicillium brasilianum (GenBank: CEJ55334.1) is numbered as TA6, the transaminase derived from Enterobacter sp.TL3 (NCBI Reference Sequence: WP_014885677.1) is numbered as TA7, the transaminase derived from Aspergillus terreus NIH2624 (NCBI Reference Sequence: XP_001209325.1) is numbered as TA8, the transaminase derived from Exophiala spinifera (NCBI Reference Sequence: XP_016233821.1) is numbered as TA9, the transaminase derived from Deinococcus geothermalis (strain DSM 11300) (NCBI Reference Sequence: WP_011530545.1) is numbered as TA10, the transaminase derived from Geomyces destructans 20631-21 (GdTA) in E. coli (GenBank: ELR05573.1) is numbered as TA11, the transaminase derived from Pseudomonas putida KT2440 (NCBI Reference Sequence: WP_010954554.1) is numbered as TA12, the transaminase derived from Lysinibacillus sphaericus (NCBI Reference Sequence: WP_024363741.1) is numbered as TA13, the transaminase derived from Bacillus megaterium DSM 319 (NCBI Reference Sequence: WP_013082219.1) is numbered as TA14, the transaminase derived from Trichoderma harzianum (GenBank: KKP07030.1) is numbered as TA15, the transaminase derived from Aspergillus fumigatus R-ATAs (AspFum) (NCBI Reference Sequence: XP_748821.1) is numbered as TA16, the transaminase derived from Geobacillus thermodenitrificans subsp.thermodenitrificans DSM 465 (NCBI Reference Sequence: WP_008879436.1) is numbered as TA17, the transaminase derived from Cladophialophora bantiana CBS 173.52 (NCBI Reference Sequence: XP_016617948.1) is numbered as TA18, the transaminase derived from Bacillus megaterium (NCBI Reference Sequence: WP_016763026.1) is numbered as TA19, the transaminase derived from Burkholderia thailandensis MSMB121 (BtS-TA) (GenBank: AGK49399.1 ) is numbered as TA20, the transaminase derived from Klebsiella pneumoniae subsp.pneumoniae MGH 78578 (NCBI Reference Sequence: WP_002920226.1) is numbered as TA21, the transaminase derived from Geobacillus toebii (NCBI Reference Sequence: WP_062753894.1) is numbered as TA22, the transaminase derived from Talaromyces cellulolyticus (GenBank: GAM37533.1) is numbered as TA23, mutants TA1-V1 (a corresponding sequence is SEQ ID NO: 1), TA1-V2 (a corresponding sequence is SEQ ID NO: 2), TA1-V3 (a corresponding sequence is SEQ ID NO: 3), and TA1-V4 (a corresponding sequence is SEQ ID NO: 4) obtained by evolution of the transaminase derived from Chromobacterium violaceum DSM30191 (CVTA) (NCBI Reference Sequence: WP_011135573.1) perform a 10 mg-level screening reaction, and an operation is as follows.

100 mmol/L phosphate buffer and 5 mol/L isopropylamine hydrochloride solution are prepared into 50 ml of solution according to a volume ratio of 5:2, and then 0.005 g of pyridoxal phosphate is added, and it is stirred and mixed uniformly. 0.3 ml of the above solution is taken and added to a 96-well plate respectively, and 10 mg 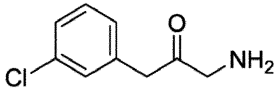 is added, and then 0.2 ml of the above enzyme solution (10 wt, the concentration of the enzyme solution is 0.5 g/ml) is added respectively, the rotation speed of a shaker is 170 rpm, the temperature is raised to 30°C and it is reacted overnight.

Screening results are shown in Fig. 4: it is proved that all the enzymes from all sources have the catalytic activity on the substrate, the transaminase derived from Chromobacterium violaceum DSM30191 (CVTA) is the best, and the transaminase derived from Chromobacterium violaceum DSM30191 (CVTA) is mutated, and four mutants obtained have the better conversion rates, and the conversion rates are all greater than 99%.

From the above descriptions, it may be seen that the above embodiments of the present invention achieve the following technical effects.
1) The use of the transaminase for the biotransformation reaction may directly obtain the desired target compound by the one-step reaction, and the substrate types are widely applicable. It not only synthesizes the compounds disclosed in the patent, but also synthesizes 1,3-diamine, 1,4-diamine and other compounds.
2) The use of the transaminase for this biotransformation reaction has the high selectivity, the chiral purity of the product is improved, and the ee value of the product is greatly improved.
3) The self-extracted transaminase is used, the substrate concentration may reach 100 g/L, and the production efficiency is greatly improved.
4) The use of the transaminase for biocatalysis does not require the use of heavy metal catalysts, azide compounds and cyanation reagents. The green chemistry is achieved.
5) The transaminase is high in catalytic efficiency, small in reaction volume, short in synthesis route, and high product yield, the three wastes are greatly reduced, and the production cost is saved.

The above are only preferred embodiments of the present invention, and are not intended to limit the present invention. For those skilled in the art, the present invention may have various modifications and changes. Any modifications, equivalent replacements, improvements and the like made within the spirit and principle of the present invention shall be included within a scope of protection of the present invention.

## Claims

1. A method for synthesizing a chiral diamine compound, comprising:
using a transaminase to convert a substrate indicated by Formula I into the chiral diamine compound; 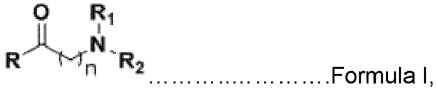
wherein n=1~10,
the R group represents an alkyl, a cycloalkyl, a heteroatom-containing alkyl, a heteroatom-containing cycloalkyl, a heteroatom-containing aryl, an amide compound residue, or an ether compound residue, wherein the heteroatom is at least one of O, S and N;
R1 and R2 are the same or different, and R1 and R2 are each independently hydrogen, a C1-C3 alkyl or an amino protecting group;
the substrate is selected from any one of the group consisting of: 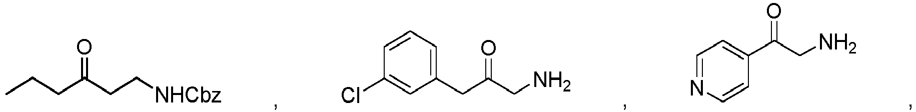 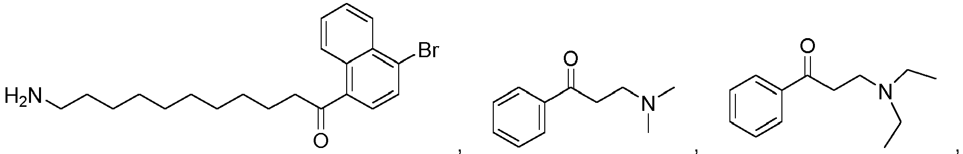 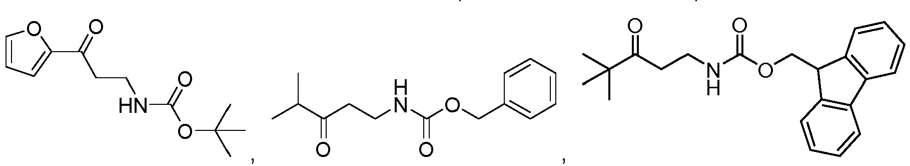 and 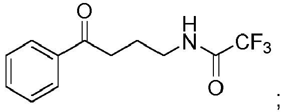
the transaminase is derived from Chromobacterium violaceum DSM30191(CVTA), Fonsecaea pedrosoi CBS 271.37, Klebsiella pneumoniae subsp. pneumoniae Ec18, Mycobacterium goodii, Paracoccus denitrificans, Penicillium brasilianum, Enterobacter sp. TL3, Aspergillus terreus NIH2624, Exophiala spinifera, Deinococcus geothermalis (strain DSM 11300), Geomyces destructans 20631-21 (GdTA) in E. coli, Pseudomonas putida KT2440, Lysinibacillus sphaericus, Bacillus megaterium DSM 319, Trichoderma harzianum, Aspergillus fumigatus R-ATAs (AspFum), Geobacillus thermodenitrificans subsp. thermodenitrificans DSM 465, Cladophialophora bantiana CBS 173.52, Bacillus megaterium, Burkholderia thailandensis MSMB121 ( BtS -TA ), Klebsiella pneumoniae subsp. pneumoniae MGH 78578, Geobacillus toebii, and Talaromyces cellulolyticus.

2. The method according to claim 1, wherein the transaminase has the amino acid sequence of SEQ ID NO: 1, SEQ ID NO: 2, SEQ ID NO: 3 or SEQ ID NO: 4.

3. The method according to claim 1, wherein the amino protecting group is selected from any one of the group consisting of tert-butoxycarbonyl, benzyloxycarbonyl, formyl, trifluoroacetyl, benzyl, trityl and 9-fluorenylmethoxycarbonyl.

4. The method according to claim 1, wherein the method comprises:
mixing a phosphate buffer with an amino donor to obtain a first mixed solution;
adding the substrate indicated by Formula I to the first mixed solution to obtain a second mixed solution;
adding the transaminase to the second mixed solution to obtain a reaction mixture;
isolating the chiral diamine compound from the reaction mixture.

5. The method according to claim 4, wherein before adding the substrate to the first mixed solution, the method further comprises: adjusting a pH value of the first mixed solution to 7.0-9.0.

6. The method according to claim 5, wherein adding the transaminase to the second mixed solution, and adjusting a pH value of the second mixed solution after addition of the transaminase to 7.0-9.0 to obtain the reaction mixture.

7. The method according to claim 6, wherein isolating the chiral diamine compound from the reaction mixture comprises:
adjusting the acidity of the reaction mixture to denature the transaminase to obtain a denatured transaminase; filtering and removing the denatured transaminase to obtain a preliminary filtrate;
adjusting a pH value of the preliminary filtrate to alkaline to obtain an alkaline filtrate;
extracting the alkaline filtrate to obtain the chiral diamine compound.

8. The method according to claim 7, wherein adjusting a pH value of the reaction mixture to 1-2.

9. The method according to claim 7, wherein a pH value of the alkaline filtration is 12-13.

10. The method according to claim 7, wherein the extraction is performed for multiple times.

11. The method according to claim 7, wherein the extraction is performed for 2 to 5 times.

12. The method according to claim 7, wherein the first extraction is performed with dichloromethane, the remaining extractions is performed with the dichloromethane or ethyl acetate.

13. The method according to claim 7, wherein after the extractions, a step of drying organic phases resulting from the extractions is further included.

14. The method according to claim 7, wherein combining the organic phases obtained from the extractions to obtain an extract;
drying the extract to obtain a dried organic phase of product;
concentrating the dried organic phase of product to no fraction under a temperature less than 45 °C and a pressure no more than - 0.08 Mpa to obtain the chiral diamine compound.

15. The method according to any one of claims 1 to 14, wherein a mass ratio of the transaminase to the substrate is 0.4:1 to 1.0:1.

16. The method according to any one of claims 1 to 14, wherein a concentration of the substrate is 60g / L to 100g / L.

17. The method according to claim 4, wherein the amino donor is selected from any one of the group consisting of isopropylamine, isopropylamine hydrochloride, alanine, phenethylamine and n-butylamine.
